(19) Europäisches Patentamt
European Patent Office
Office européen des brevets

(11) Veröffentlichungsnummer: **0 484 750 A1**

# (12) EUROPÄISCHE PATENTANMELDUNG

(21) Anmeldenummer: **91118130.3**

(22) Anmeldetag: **24.10.91**

(51) Int. Cl.5: **C07D 403/04**, A01N 43/653, //(C07D403/04,249:00,239:00)

(30) Priorität: **06.11.90 DE 4035141**

(43) Veröffentlichungstag der Anmeldung: **13.05.92 Patentblatt 92/20**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Kirsten, Rolf, Dr.**
**Carl-Langhans-Strasse 27**
**W-4019 Monheim(DE)**
Erfinder: **Wolf, Hilmar, Dr.**
**Zum Bräuhaus 14**
**W-4018 Langenfeld(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen(DE)**
Erfinder: **Schmidt, Robert R., Dr.**
**Im Waldwinkel 110**
**W-5060 Bergisch Gladbach 2(DE)**

(54) **Substituierte Sulfonylaminotriazolylpyrimidine.**

(57) Die Erfindung betrifft neue substituierte Sulfonylaminotriazolylpyrimidine der Formel (I),

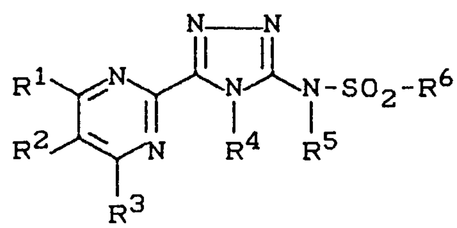

(I)

in welcher

| | |
|---|---|
| $R^1$, $R^2$ und $R^3$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können, |
| $R^4$ | für Wasserstoff oder für jeweils gegebenenfalls substituiertes Alkyl, Alkenyl, Alkinyl, Aralkyl oder Aryl steht, |
| $R^5$ | für Wasserstoff, ein Ammonium-, Alkylammonium-, Dialkylammonium- oder Trialkylammonium-äquivalent oder ein Metalläquivalent steht und |
| $R^6$ | für jeweils gegebenenfalls substituiertes Aralkyl, Aryl oder Heteroaryl steht, |

ein Verfahren und neue Zwischenprodukte zu ihrer Herstellung sowie ihre Verwendung als Herbizide.

EP 0 484 750 A1

Die Erfindung betrifft neue substituierte Sulfonylaminotriazolylpyrimidine, ein Verfahren und neue Zwischenprodukte zu ihrer Herstellung sowie ihre Verwendung als Herbizide.

Es ist bekannt, daß 3-Amino-1,2,4-triazol (Amitrol) als Herbizid verwendet werden kann (vgl. Science 145 (1964), 97). Die Wirkung dieser Verbindung ist jedoch nicht in allen Belangen zufriedenstellend.

Weiter sind bestimmte substituierte Sulfonylaminoazole mit herbiziden Eigenschaften bekanntgeworden (vgl. DE-OS 3 737 748 und DE-OS 3 825 867). Verbindungen aus den genannten Publikationen haben jedoch bisher keine größere Bedeutung erlangt.

Es wurden nun die neuen substituierten Sulfonylaminotriazolylpyrimidine der allgemeinen Formel (I) gefunden,

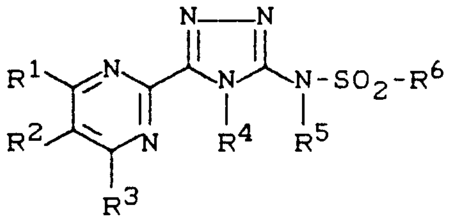

(I)

in welcher

- $R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,
- $R^4$ für Wasserstoff oder für jeweils gegebenenfalls substituiertes Alkyl, Alkenyl, Alkinyl, Aralkyl oder Aryl steht,
- $R^5$ für Wasserstoff, ein Ammonium-, Alkylammonium-, Dialkylammonium- oder Trialkylammonium-äquivalent oder ein Metalläquivalent steht und
- $R^6$ für jeweils gegebenenfalls substituiertes Aralkyl, Aryl oder Heteroaryl steht.

Man erhält die neuen substituierten Sulfonylaminotriazolylpyrimidine der allgemeinen Formel (I), wenn man Thiosemicarbazid-Derivate der allgemeinen Formel (II)

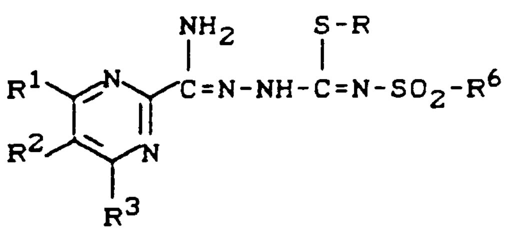

(II)

in welcher

- $R^1$, $R^2$, $R^3$ und $R^6$ die oben angegebene Bedeutung haben und
- R für Alkyl steht,

und/oder Tautomere der Verbindungen der Formel (II) mit Aminen der allgemeinen Formel (III)

$H_2N-R^4$

in welcher

- $R^4$ die oben angegebene Bedeutung hat,

gegebenenfalls in Gegenwart eines Verdünnungsmittels umsetzt und gegebenenfalls die so erhaltenen Verbindungen der Formel (I), in welcher $R^5$ für Wasserstoff steht, durch Umsetzung mit Ammoniak, Alkyl-, Dialkyl- oder Trialkylaminen oder mit geeigneten Metallverbindungen nach üblichen Methoden in entsprechende Salze überführt.

Die neuen substituierten Sulfonylaminotriazolylpyrimidine der allgemeinen Formel (I) zeichnen sich durch starke herbizide Wirksamkeit aus. Überraschenderweise zeigen die neuen Verbindungen der Formel (I) erheblich bessere herbizide Wirkung als das strukturell ähnliche bekannte Herbizid Aminotriazol (Amitrol).

Bevorzugte Substituenten werden im folgenden erläutert: Alkyl steht in den allgemeinen Formeln einzeln oder in zusammengesetzten Resten, wie z.B. Alkoxy, Alkylthio, Alkylamino, jeweils für geradkettiges oder verzweigtes Alkyl, vorzugsweise mit 1 bis 6, insbesondere mit 1 bis 4 Kohlenstoffatomen. Beispielhaft seien genannt: Methyl, Ethyl, n- und iso-Propyl, n-, iso-, sec- und tert-Butyl.

Alkenyl und Alkinyl stehen jeweils für geradkettiges oder verzweigtes Alkenyl oder Alkinyl mit vorzugsweise 2 bis 6, insbesondere 3 bis 5 Kohlenstoffatomen. Beispielhaft seien genannt: Allyl und Propargyl.

Halogenalkyl steht in den allgemeinen Formeln einzeln oder in zusammengesetzten Resten, wie z.B. Halogenalkoxy, jeweils für geradkettiges oder verzweigtes Halogenalkyl, vorzugsweise mit 1 bis 6 Kohlenstoffatomen und 1 bis 13 Halogenatomen, vorzugsweise Fluor- oder Chloratomen, insbesondere mit 1 bis 4 Kohlenstoffatomen und 1 bis 9 Halogenatomen. Beispielhaft seien genannt: Fluormethyl, Chlormethyl, Difluormethyl, Dichlormethyl, Trifluormethyl, Trichlormethyl, Chlorfluormethyl, Chlordifluormethyl, Fluordichlormethyl, Fluorethyl, Difluorethyl, Trifluorethyl, Tetrafluorethyl, Chlorethyl, Dichlorethyl, Trichlorethyl, Chlorfluorethyl, Chlordifluorethyl, Chlortrifluorethyl, Fluorpropyl, Chlorpropyl, Fluorbutyl und Chlorbutyl.

Aryl steht in den allgemeinen Formeln einzeln oder in zusammengesetzten Resten, wie Aralkyl, jeweils für Phenyl oder Naphthyl, vorzugsweise für Phenyl.

Heteroaryl steht in den allgemeinen Formeln vorzugsweise für fünfgliedrige oder sechsgliedrige, gegebenenfalls benzanellierte aromatische Heterocyclen mit bis zu 3 Stickstoffatomen und/oder gegebenenfalls einem Sauerstoff- oder Schwefelatom, insbesondere für Thienyl, Pyrazolyl oder Pyridyl.

Die Erfindung betrifft vorzugsweise Verbindungen der Formel (I), in welcher

$R^1$ für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_2$-Alkoxy-$C_1$-$C_2$-alkoxy, $C_1$-$C_4$-Alkylthio, $C_1$-$C_4$-Alkylamino oder Di-($C_1$-$C_2$-alkyl)-amino steht,

$R^2$ für Wasserstoff, Halogen, $C_1$-$C_4$-Alkyl, $C_1$-$C_4$-Halogenalkyl oder $C_1$-$C_4$-Alkoxy steht oder zusammen mit $R^1$ oder $R^3$ für Trimethylen oder Tetramethylen steht,

$R^3$ für Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

$R^4$ für Wasserstoff oder für jeweils gegebenenfalls durch Halogen substituiertes $C_1$-$C_6$-Alkyl, $C_2$-$C_6$-Alkenyl oder $C_2$-$C_6$-Alkinyl oder für jeweils gegebenenfalls durch Halogen, Cyano, Carboxy, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkylsulfinyl, $C_1$-$C_2$-Halogenalkylsulfinyl, $C_1$-$C_4$-Alkylsulfonyl, $C_1$-$C_2$-Halogenalkylsulfonyl und/oder $C_1$-$C_4$-Alkoxy-carbonyl substituiertes Phenyl oder Benzyl steht,

$R^5$ für Wasserstoff, ein Ammoniumion, ein $C_1$-$C_6$-Alkylammoniumion, ein Di-($C_1$-$C_6$-alkyl)-ammoniumion, ein Tri-($C_1$-$C_4$-alkyl)-ammoniumion, ein Lithium-, Natrium- oder Kaliumion oder ein Magnesium- oder Calciumäquivalent steht, und

$R^6$ für den Rest

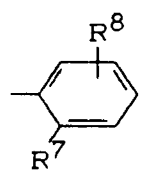

steht, worin

$R^7$ und $R^8$ gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)aminocarbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor,

Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxycarbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest -$S(O)_p$-$R^9$ stehen, wobei

p für die Zahlen 1 oder 2 steht und

$R^9$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest -NHOR$^{10}$ steht, wobei

$R^{10}$ für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^7$ und $R^8$ weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonylamino, Di-($C_1$-$C_4$-alkyl)-amino-carbonylamino, oder für den Rest -CO-$R^{11}$ stehen, wobei

$R^{11}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^7$ und $R^8$ weiterhin für $C_1$-$C_4$-Alkylsulfonyloxy, Di-($C_1$-$C_4$-alkyl)-aminosulfonylamino oder für den Rest -CH = N-$R^{12}$ stehen, wobei

$R^{12}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

weiter

$R^6$ für den Rest

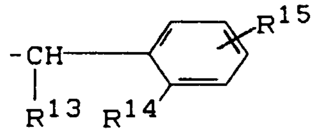

steht, worin

$R^{13}$ für Wasserstoff oder $C_1$-$C_4$-Alkyl steht,

$R^{14}$ und $R^{15}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Carboxy, $C_1$-$C_4$-Alkoxy-carbonyl, $C_1$-$C_4$-Alkylsulfonyl oder Di-($C_1$-$C_4$-alkyl)-aminosulfonyl stehen;

weiter

$R^6$ für den Rest

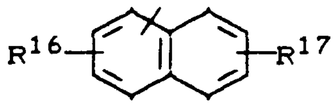

steht, worin

$R^{16}$ und $R^{17}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

weiter

$R^6$ für den Rest

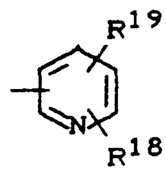

steht, worin

$R^{18}$ und $R^{19}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylaminocarbonyl oder Dioxolanyl stehen;

weiter

$R^6$ für den Rest

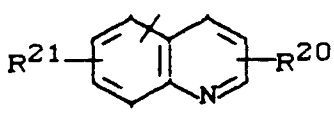

steht, worin

$R^{20}$ und $R^{21}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

weiter

$R^6$ für den Rest

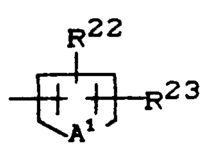

steht, worin

$R^{22}$ und $R^{23}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl

(welches gegebenenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

weiter

$R^6$ für den Rest

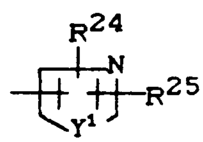

steht, worin

$R^{24}$ und $R^{25}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{26}$ steht, wobei

$R^{26}$ für Wasserstoff oder $C_1$-$C_4$-Alkyl steht;

weiter

$R^6$ für den Rest

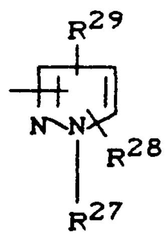

steht, worin

$R^{27}$ für Wasserstoff, $C_1$-$C_4$-Alkyl, Phenyl oder (Iso)Chinolinyl steht,

$R^{28}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{29}$ für Wasserstoff, Halogen oder $C_1$-$C_4$-Alkyl steht;

weiter

$R^6$ für den Rest

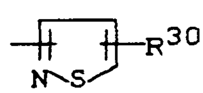

steht, worin

$R^{30}$ für Wasserstoff, Halogen, $C_1$-$C_4$-Alkyl oder $C_1$-$C_4$-Alkoxy-carbonyl steht.

Die Erfindung betrifft insbesondere Verbindungen der Formel (I), in welcher

$R^1$ für Wasserstoff, Fluor, Chlor, Brom, Methyl, Ethyl, Trifluormethyl, Chlormethyl, Methoxethyl, Methoxy, Ethoxy, Difluormethoxy, 2,2,2-Trifluorethoxy, 2-Methoxy-ethoxy, Methylthio, Methylamino, Ethylamino oder Dimethylamino steht,

$R^2$ für Wasserstoff, Fluor, Chlor, Brom, Methyl, Trifluormethyl oder Methoxy steht,

$R^3$ für Wasserstoff, Fluor, Chlor, Brom, Methyl, Ethyl, Trifluormethyl, Chlormethyl, Methoxy, Ethoxy

oder Difluormethoxy steht,

$R^4$ für Wasserstoff, Methyl, Ethyl, Propyl, Isopropyl, Butyl, Isobutyl, sec-Butyl, tert-Butyl, Allyl, Propargyl, Phenyl oder Benzyl steht,

$R^5$ für Wasserstoff steht und

$R^6$ für den Rest

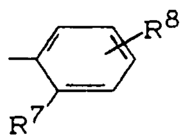

steht, worin

$R^7$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, N-Methoxy-N-methylaminosulfonyl, Phenyl, Phenoxy oder $C_1$-$C_3$-Alkoxy-carbonyl steht und

$R^8$ für Wasserstoff, Fluor oder Chlor steht;

weiter

$R^6$ für den Rest

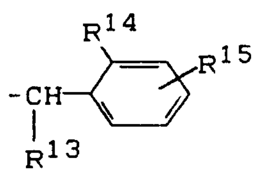

steht, worin

$R^{13}$ für Wasserstoff steht,

$R^{14}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{15}$ für Wasserstoff, Fluor oder Chlor steht;

weiter

$R^6$ für den Rest

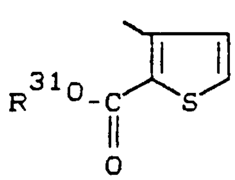

steht, worin

$R^{31}$ für Methyl oder Ethyl steht, oder

$R^6$ für den Rest

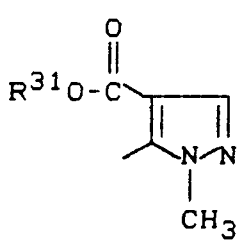

steht, worin

$R^{31}$ für Methyl oder Ethyl steht.

Verwendet man beispielsweise 1-(1-Amino-1-(4,6-dimethylpyrimidin-2-yl)-methylen)-4-(2-fluor-phenylsulfonyl)-S-methyl-isothiosemicarbazid und Ethylamin als Ausgangsstoffe, so kann der Reaktionsablauf beim erfindungsgemäßen Verfahren durch das folgende Formelschema skizziert werden:

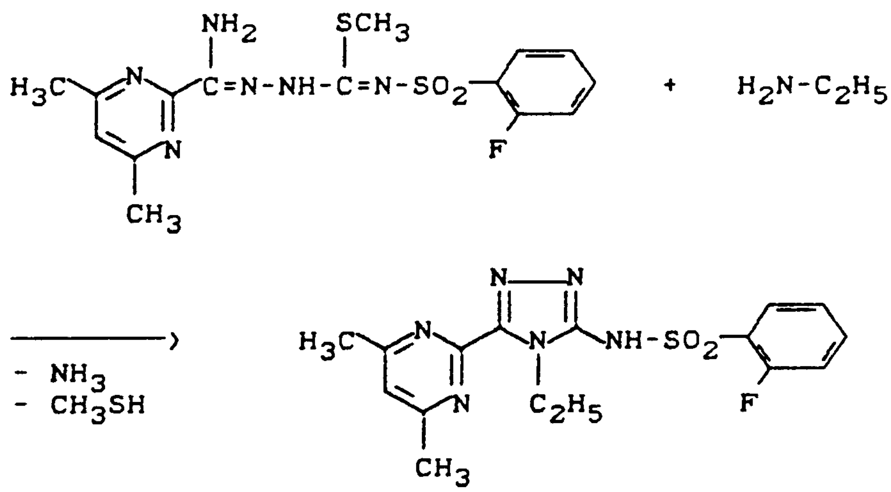


Die beim erfindungsgemäßen Verfahren zur Herstellung von Verbindungen der Formel (I) als Ausgangsstoffe zu verwendenden Thiosemicarbazid-Derivate sind durch die Formel (II) allgemein definiert.

In Formel (II) haben $R^1$, $R^2$, $R^3$ und $R^6$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$ und $R^6$ angegeben wurden;

R steht vorzugsweise für $C_1$-$C_4$-Alkyl, insbesondere für Methyl.

Beispiele für die Ausgangsstoffe der Formel (II) sind in der nachstehenden Tabelle 1 aufgeführt.

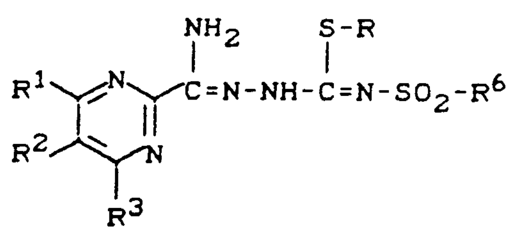

(II)

Tabelle 1: Beispiele für die Verbindungen der Formel (II)

| R | $R^1$ | $R^2$ | $R^3$ | $R^6$ |
|---|---|---|---|---|
| $CH_3$ | $CH_3$ | H | H | 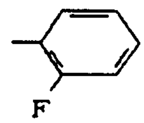 |
| $CH_3$ | $CH_3$ | H | $CH_3$ | 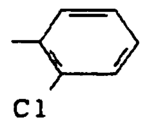 |
| $CH_3$ | $OCH_3$ | H | $CH_3$ | 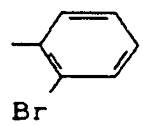 |
| $CH_3$ | $CH_3$ | F | $CH_3$ | 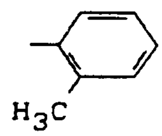 |
| $CH_3$ | $CH_3$ | H | $CH_3$ | 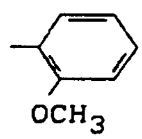 |
| $CH_3$ | $OCH_3$ | H | $OCH_3$ | 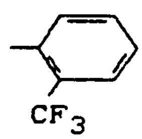 |
| $CH_3$ | $OCH_3$ | H | $OCH_3$ | 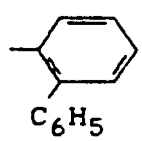 |

Tabelle 1 - Fortsetzung

| R | $R^1$ | $R^2$ | $R^3$ | $R^6$ |
|---|---|---|---|---|
| $CH_3$ | $OCH_3$ | H | $OCH_3$ | 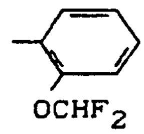 |
| $CH_3$ | $OCH_3$ | H | $OCH_3$ | 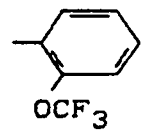 |
| $CH_3$ | $OCH_3$ | H | $CH_3$ | 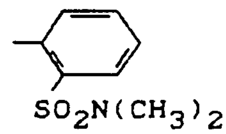 |
| $CH_3$ | $OCH_3$ | H | $CF_3$ | 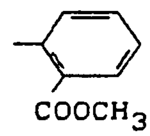 |
| $CH_3$ | H | $CH_3$ | H | 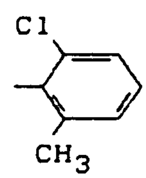 |
| $C_2H_5$ | H | $OCH_3$ | H | 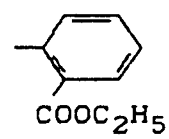 |
| $CH_3$ | $CH_3$ | $CH_3$ | $CH_3$ | 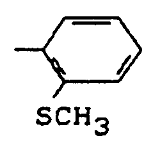 |
| $CH_3$ | $OCH_3$ | H | $OCH_3$ | 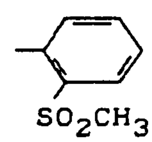 |

Tabelle 1 - Fortsetzung

| R | $R^1$ | $R^2$ | $R^3$ | $R^6$ |
|---|---|---|---|---|
| $CH_3$ | $OCH_3$ | H | $OCH_3$ | 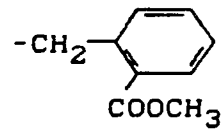 |
| $CH_3$ | $OCH_3$ | H | $CH_3$ | 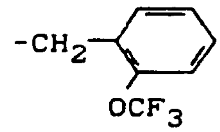 |
| $CH_3$ | $OCH_3$ | H | $CH_3$ | 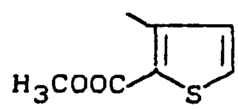 |
| $CH_3$ | $OCH_3$ | H | $OCH_3$ | 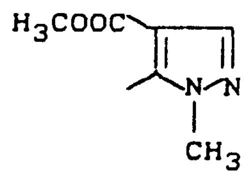 |

Die Ausgangsstoffe der Formel (II) sind noch nicht aus der Literatur bekannt und sind ebenfalls Gegenstand der vorliegenden Anmeldung.

Man erhält die neuen Thiosemicarbazid-Derivate der allgemeinen Formel (II), wenn man Amidrazone der allgemeinen Formel (IV)

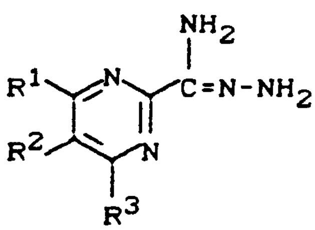

(IV)

in welcher
$R^1$, $R^2$ und $R^3$ die oben angegebene Bedeutung haben,
mit Sulfonyliminodithiokohlensäurediestern der allgemeinen Formel (V)

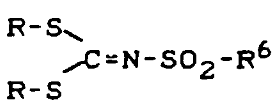

(V)

in welcher
R und $R^6$ die oben angegebene Bedeutung haben,
gegebenenfalls in Gegenwart eines Verdünnungsmittels, wie z.B. Tetrahydrofuran, bei Temperaturen zwischen 0 °C und 150 °C, vorzugsweise zwischen 20 °C und 100 °C, umsetzt und nach üblichen Methoden aufarbeitet (vgl. die Herstellungsbeispiele).

Die als Zwischenprodukte benötigten Amidrazone sind durch die Formel (IV) allgemein definiert. In Formel (IV) haben $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden.

Als Beispiele für die Verbindungen der Formel (IV) seien genannt:
Pyrimidin-2-carbamidrazon, 4-Methyl-, 4,6-Dimethyl, 5-Methyl-, 4-Methoxy-6-methyl-, 5-Methoxy-, 4,6-Dimethoxy-, 4-Methoxy-6-trifluormethyl-, 4-Methoxy-6-ethyl-, 4-Ethoxy-6-methyl- und Bis-4,6-difluormethoxy-pyrimidin-2-carbamidrazon.

Die Amidrazone der Formel (IV) sind bekannt und/oder können nach an sich bekannten Verfahren hergestellt werden (vgl. J. Org. Chem. 30 (1965), 931-933; Herstellungsbeispiele).

Die weiter als Zwischenprodukte benötigten Sulfonyliminodithiokohlensäurediester sind durch die Formel (V) allgemein definiert. In Formel (V) hat $R^6$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^6$ angegeben wurde; R steht vorzugsweise für $C_1$-$C_4$-Alkyl, insbesondere für Methyl.

Die Sulfonyliminodithiokohlensäurediester der Formel (V) sind bekannt und/oder können nach an sich bekannten Verfahren hergestellt werden; Beispiele für diese Verbindungen und für ihre Herstellung können den angegebenen Publikationen entnommen werden (vgl. EP-A 173 321, DE-OS 3 726 424).

Die beim erfindungsgemäßen Verfahren zur Herstellung von Verbindungen der Formel (I) weiter als Ausgangsstoffe zu verwendenden Amine sind durch die Formel (III) allgemein definiert.

In Formel (III) hat $R^4$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^4$ angegeben wurde.

Als Beispiele für die Ausgangsstoffe der Formel (III) seien genannt:
Methylamin, Ethylamin, Propylamin, Isopropylamin, Butylamin, Isobutylamin, Allylamin, Propargylamin, Benzylamin und Anilin.

Die Ausgangsstoffe der Formel (III) sind bekannte Synthesechemikalien.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol, Propanol, Isopropanol, Butanol, Iso- und sec-Butanol, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Thiosemicarbazid-Derivat der Formel (II) im allgemeinen zwischen 1 und 200 Mol, vorzugsweise zwischen 2 und 100 Mol, Amine der Formel (III) ein.

Die Reaktionskomponenten werden im allgemeinen bei Raumtemperatur vermischt und das Reaktionsgemisch wird dann, vorzugsweise bei erhöhter Temperatur, bis zum Ende der Umsetzung gerührt. Die Aufarbeitung kann nach üblichen Methoden durchgeführt werden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[[(4,6-Dimethoxypyrimidin-2-yl)-aminol-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino)-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxyl-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxyl-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxyl-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,-5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[-(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitrophenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octylthiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN).

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 2 g und 2 kg pro ha.

Die Herstellung und die Verwendung der erfindungsgemäßen Wirkstoffe geht aus den nachfolgenden Beispielen hervor.

Herstellungsbeispiele:

Beispiel 1

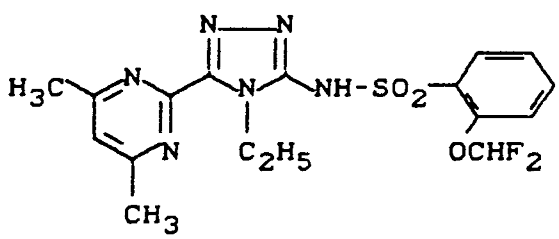

2,2 g (5 mMol) 1-(1-Amino-1-(4,6-dimethyl-pyrimidin-2-yl)-methylen)-4-(2-difluormethoxy-phenylsulfonyl)-S-methyl-isothiosemicarbazid werden mit 80 ml 23%iger ethanolischer Ethylamin-Lösung verrührt und die Mischung wird 24 Stunden unter Rückfluß erhitzt. Dann wird eingeengt, der Rückstand durch Verreiben mit Diethylether zur Kristallisation gebracht und das kristalline Produkt durch Absaugen isoliert.

Man erhält 1,8 g (77% der Theorie) 3-(4,6-Dimethyl-pyrimidin-2-yl)-4-ethyl-5-(2-difluormethoxy-phenylsulfonylamino)-1,2,4-triazol-ethylammoniumsalz vom Schmelzpunkt 212 °C. Durch Verrühren dieses Produkts mit 15 ml 1N-Salzsäure, Absaugen und Trocknen erhält man das freie 3-(4,6-Dimethyl-pyrimidin-2-yl)-4-ethyl-5-(2-difluormethoxy-phenylsulfonylamino)-1,2,4-triazol vom Schmelzpunkt 217 °C (Ausbeute: 75% der Theorie).

Analog Beispiel 1 und entsprechend der allgemeinen Beschreibung des erfindungsgemäßen Herstellungsverfahrens können beispielsweise auch die in der nachstehenden Tabelle 2 aufgeführten Verbindungen der Formel (I) hergestellt werden.

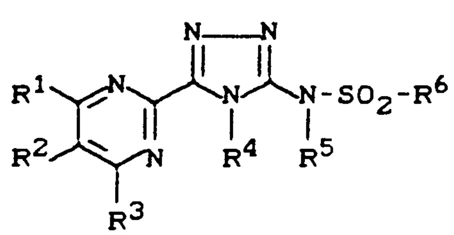

(I)

Tabelle 2: Beispiele für die Verbindungen der Formel (I)

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 2 | $OCH_3$ | H | $OCH_3$ | $CH_3$ | H | 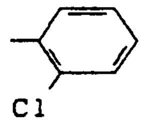 | 175 |
| 3 | $OCH_3$ | H | $OCH_3$ | $CH_3$ | H | 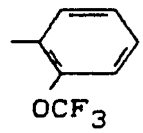 | 154 |
| 4 | $OCH_3$ | H | $OCH_3$ | $CH_3$ | H | 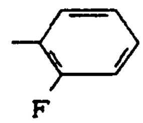 | 257 |
| 5 | $OCH_3$ | H | $OCH_3$ | $C_2H_5$ | H | 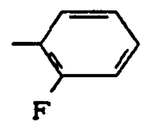 | 170 |
| 6 | $OCH_3$ | H | $OCH_3$ | $CH(CH_3)_2$ | H | 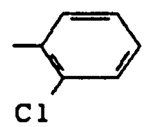 | 240 |

Tabelle 2 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 7 | $OCH_3$ | H | $OCH_3$ | $C_2H_5$ | H | 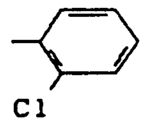 | 195 |
| 8 | $OCH_3$ | H | $OCH_3$ | $C_3H_7$-n | H | 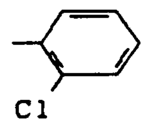 | 240 |
| 9 | $OCH_3$ | H | $OCH_3$ | $CH_3$ | H | 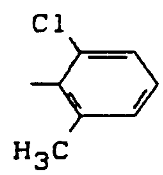 | 210 |
| 10 | $OCH_3$ | H | $OCH_3$ | $C_2H_5$ | H | 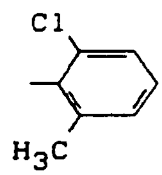 | 236 |
| 11 | $OCH_3$ | H | $OCH_3$ | $C_3H_7$-n | H | 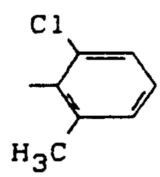 | 259 |
| 12 | $OCH_3$ | H | $OCH_3$ | $CH_3$ | H | 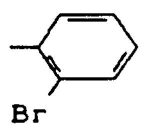 | 214 |
| 13 | $OCH_3$ | H | $OCH_3$ | $CH_3$ | H | 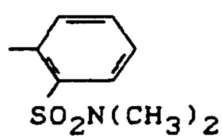 | 122 |
| 14 | $OCH_3$ | H | $OCH_3$ | $C_2H_5$ | H | 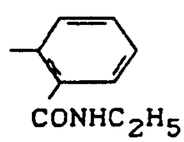 | |

<u>Tabelle 2</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 15 | $OCH_3$ | H | $OCH_3$ | $C_2H_5$ | H | 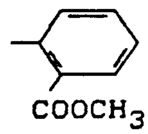 | |
| 16 | $OCH_3$ | H | $OCH_3$ | $C_3H_7$-n | H | 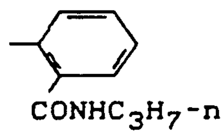 | 78 |
| 17 | $CH_3$ | H | $CH_3$ | $CH_3$ | H | 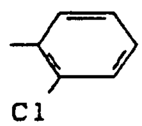 | 300 |
| 18 | $CH_3$ | H | $CH_3$ | $CH_3$ | H | 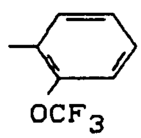 | 211 |
| 19 | $CH_3$ | H | $CH_3$ | $CH_3$ | H | 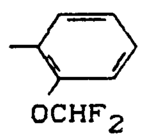 | 223 |
| 20 | $CH_3$ | H | $CH_3$ | $CH_3$ | H | 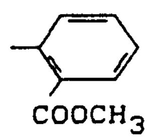 | 198 |
| 21 | $CH_3$ | H | $CH_3$ | $C_2H_5$ | H | 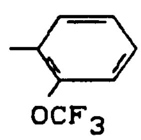 | 216 |
| 22 | $CH_3$ | H | $CH_3$ | $CH_3$ | H | 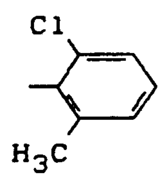 | 277 |

Tabelle 2 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 23 | $CH_3$ | H | $CH_3$ | $CH_3$ | H | 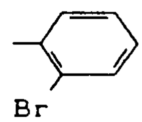 | 296 |
| 24 | $CH_3$ | H | $CH_3$ | $CH_3$ | H | 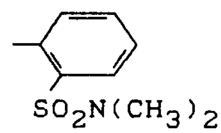 | 110 |
| 25 | $CH_3$ | H | $OCH_3$ | $C_2H_5$ | H | 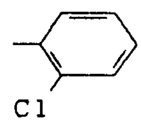 | 203 |
| 26 | $CH_3$ | H | $OCH_3$ | $CH_3$ | H | 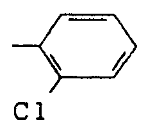 | 240 |
| 27 | $CH_3$ | H | $OCH_3$ | $CH_3$ | H | 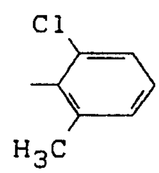 | 211 |
| 28 | $CH_3$ | H | $OCH_3$ | $CH_3$ | H | 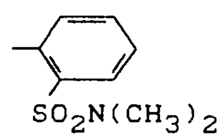 | 237 |
| 29 | $CH_3$ | H | $OCH_3$ | $CH_3$ | H | 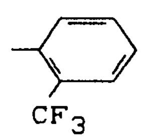 | 235 |
| 30 | $CH_3$ | H | $OCH_3$ | $CH_3$ | H | 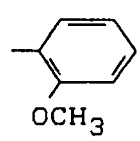 | |
| 31 | $CH_3$ | H | $CH_3$ | H | H | 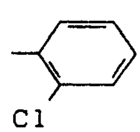 | 142 |

Ausgangsstoffe der Formel (II)

Beispiel (II-1)

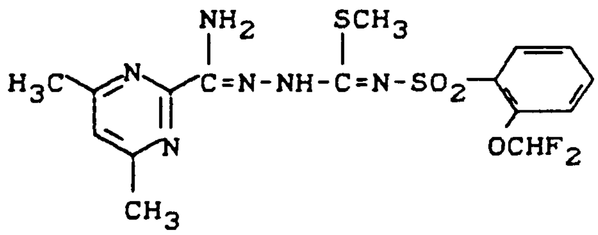

Eine Mischung aus 5,0 g (0,03 Mol) 4,6-Dimethyl-pyrimidin-2-carbamidrazon, 9,8 g (0,03 Mol) N-(2-Difluormethoxy-phenylsulfonyl)-iminodithiokohlensäure-S,S-dimethylester und 100 ml Tetrahydrofuran wird 2 Stunden unter Rückfluß erhitzt. Anschließend läßt man das Gemisch auf Raumtemperatur (ca. 20 °C) abkühlen und isoliert das kristallin angefallene Produkt durch Absaugen.

Man erhält 9,4 g (71% der Theorie) 1-(1-Amino-1-(4,6-dimethyl-pyrimidin-2-yl)-methylen)-4-(2-difluormethoxyphenylsulfonyl)-S-methyl-isothiosemicarbazid vom Schmelzpunkt 196 °C.

Ausgangsstoffe der Formel (IV):

Beispiel (IV-1)

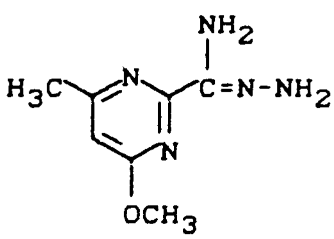

1. Stufe:

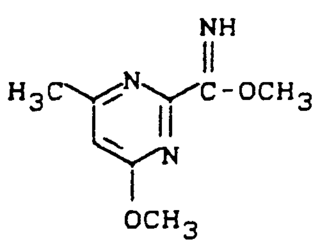

104,3 g (0,7 Mol) 4-Methyl-6-methoxy-pyrimidin-2-carbonsäurenitril werden in 600 ml Methanol gelöst und 3,8 g (0,07 Mol) festes Natriummethanolat werden zugefügt. Die Lösung wird 16 Stunden bei Raumtemperatur gerührt, mit 4 ml Eisessig versetzt und eingeengt. Der Rückstand wird mit Petrolether auskristallisiert, die Kristalle werden abgesaugt und an der Luft getrocknet.

Man erhält 110,7 g (87% der Theorie) 4-Methyl-6-methoxy-pyrimidin-2-carbonimidsäure-methylester vom Schmelzpunkt 116 °C.

2. Stufe:

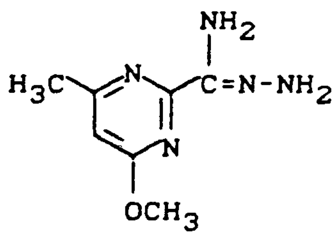

108,6 g (0,6 Mol) 4-Methyl-6-methoxy-pyrimidin-2-carbon-imidsäure-methylether werden in 500 ml Methanol gelöst. Man gibt 1 g p-Toluolsulfonsäure und 29 ml (0,6 Mol) Hydrazinhydrat zu und rührt das Reaktionsgemisch 16 Stunden bei Raumtemperatur. Der nach dem Einengen verbleibende Rückstand wird mit Diethylether zur Kristallisation gebracht. Die gelben Kristalle werden abgesaugt und bei 50 ° C getrocknet.

Man erhält 105,5 g (97% der Theorie) 4-Methyl-6-methoxy-pyrimidin-2-carbamidrazon vom Schmelz- und Zersetzungspunkt 105 ° C.

Analog erhält man:

Beispiel (IV-2)

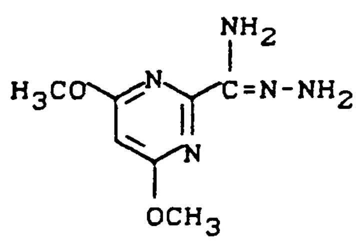

4,6-Dimethoxy-pyrimidin-2-carbamidrazon; Schmelzpunkt: 122 ° C.

Beispiel (IV-3)

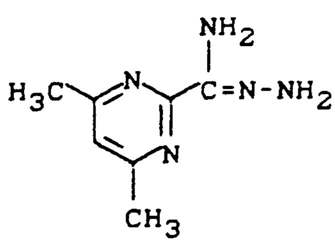

4,6-Dimethyl-pyrimidin-2-carbamidrazon; Schmelzpunkt: 84 ° C.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigt in diesem Test z.B. die Verbindung gemäß Herstellungsbeispiel 17.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen 17, 18 und 19.

## Patentansprüche

**1.** Substituierte Sulfonylaminotriazolylpyrimidine der allgemeinen Formel (I),

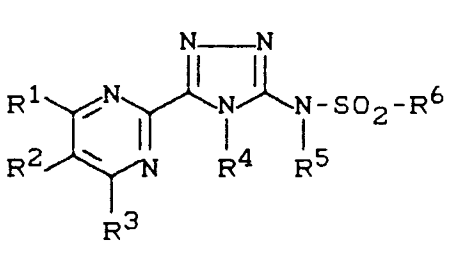

(I)

in welcher

$R^1$, $R^2$ und $R^3$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können,

$R^4$ für Wasserstoff oder für jeweils gegebenenfalls substituiertes Alkyl, Alkenyl, Alkinyl, Aralkyl oder Aryl steht,

$R^5$ für Wasserstoff, ein Ammonium-, Alkylammonium-Dialkylammonium- oder Trialkylammonium-äquivalentoder ein Metalläquivalent steht und

$R^6$ für jeweils gegebenenfalls substituiertes Aralkyl, Aryl oder Heteroaryl steht.

**2.** Substituierte Sulfonylaminotriazolylpyrimidine der allgemeinen Formel (I) gemäß Anspruch 1, dadurch gekennzeichnet, daß darin

$R^1$ für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_2$-Alkoxy-$C_1$-$C_2$-alkoxy, $C_1$-$C_4$-Alkylthio, $C_1$-$C_4$-Alkylamino oder Di-($C_1$-$C_2$-alkyl)-amino steht,

$R^2$ für Wasserstoff, Halogen, $C_1$-$C_4$-Alkyl, $C_1$-$C_4$-Halogenalkyl oder $C_1$-$C_4$-Alkoxy steht oder zusammen mit $R^1$ oder $R^3$ für Trimethylen oder Tetramethylen steht,

$R^3$ für Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy steht,

$R^4$ für Wasserstoff oder für jeweils gegebenenfalls durch Halogen substituiertes $C_1$-$C_6$-Alkyl, $C_2$-$C_6$-Alkenyl oder $C_2$-$C_6$-Alkinyl oder für jeweils gegebenenfalls durch Halogen, Cyano, Carboxy, Nitro, $C_1$-$C_4$- Alkyl, $C_1$-$C_2$-Halogenalkyl, $C_1$-$C_4$-Alkoxy, $C_1$- $C_2$-Halogenalkoxy, $C_1$-$C_4$-Alkylthio, $C_1$-$C_2$-Halogenalkylthio, $C_1$-$C_4$-Alkylsulfinyl, $C_1$-$C_2$-Halogenalkylsulfinyl, $C_1$-$C_4$-Alkylsulfonyl, $C_1$-$C_2$- Halogenalkylsulfonyl und/oder $C_1$-$C_4$-Alkoxy-carbonyl substituiertes Phenyl oder Benzyl steht,

$R^5$ für Wasserstoff, ein Ammoniumion, ein $C_1$-$C_6$-Alkylammoniumion, ein Di-($C_1$-$C_6$-alkyl)-ammoniumion, ein Tri-($C_1$-$C_4$-alkyl)-ammoniumion, ein Lithium-, Natrium- oder Kaliumion oder ein Magnesium- oder Calciumäquivalent steht, und

$R^6$ für den Rest

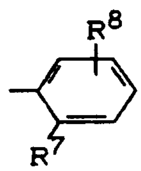

steht, worin

$R^7$ und $R^8$ gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylaminocarbonyl, Di-($C_1$-$C_4$-alkyl)-aminocarbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^9$ stehen, wobei

p für die Zahlen 1 oder 2 steht und

$R^9$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^{10}$ steht, wobei

$R^{10}$ für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^7$ und $R^8$ weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonylamino, Di-($C_1$-$C_4$-alkyl)-amino-carbonylamino, oder für den Rest -CO-$R^{11}$ stehen, wobei

$R^{11}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^7$ und $R^8$ weiterhin für $C_1$-$C_4$-Alkylsulfonyloxy, Di-($C_1$-$C_4$-alkyl)-aminosulfonylamino oder für den Rest -CH = N-$R^{12}$ stehen, wobei

$R^{12}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

weiter

$R^6$ für den Rest

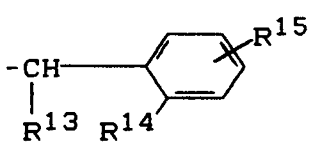

steht, worin

$R^{13}$ für Wasserstoff oder $C_1$-$C_4$-Alkyl steht,

$R^{14}$ und $R^{15}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Carboxy, $C_1$-$C_4$-Alkoxy-carbonyl, $C_1$-$C_4$-Alkylsulfonyl oder Di-($C_1$-$C_4$-alkyl)-aminosulfonyl stehen;

weiter

$R^6$ für den Rest

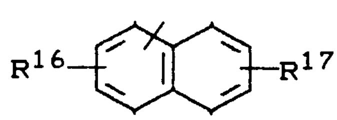

steht, worin

$R^{16}$ und $R^{17}$ gleich oder verschieden sind und für Wasserstoff, Fluor, Chlor, Brom, Nitro, Cyano,

$C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

weiter

$R^6$ für den Rest

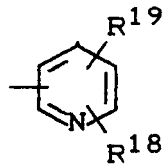

steht, worin

$R^{18}$ und $R^{19}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylaminocarbonyl oder Dioxolanyl stehen;

weiter

$R^6$ für den Rest

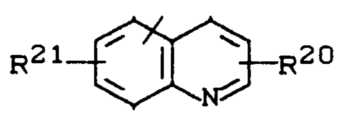

steht, worin

$R^{20}$ und $R^{21}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

weiter

$R^6$ für den Rest

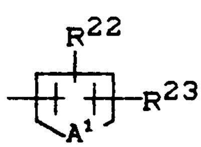

steht, worin

$R^{22}$ und $R^{23}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-aminosulfonyl oder $C_1$-$C_4$-Alkoxy-carbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder

Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

weiter

$R^6$ für den Rest

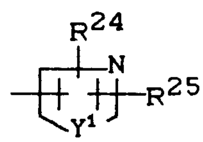

steht, worin

$R^{24}$ und $R^{25}$ gleich oder verschieden sind und für Wasserstoff, $C_1$-$C_4$-Alkyl, Halogen, $C_1$-$C_4$-Alkoxy-carbonyl, $C_1$-$C_4$-Alkoxy oder $C_1$-$C_4$-Halogenalkoxy stehen,

$Y^1$ für Schwefel oder die Gruppierung N-$R^{26}$ steht, wobei

$R^{26}$ für Wasserstoff oder $C_1$-$C_4$-Alkyl steht;

weiter

$R^6$ für den Rest

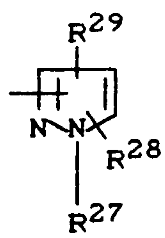

steht, worin

$R^{27}$ für Wasserstoff, $C_1$-$C_4$-Alkyl, Phenyl oder (Iso)Chinolinyl steht,

$R^{28}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{29}$ für Wasserstoff, Halogen oder $C_1$-$C_4$-Alkyl steht;

weiter

$R^6$ für den Rest

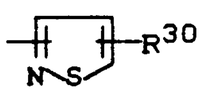

steht, worin

$R^{30}$ für Wasserstoff, Halogen, $C_1$-$C_4$-Alkyl oder $C_1$-$C_4$-Alkoxy-carbonyl steht.

**3.** Substituierte Sulfonylaminotriazolylpyrimidine der allgemeinen Formel (I) gemäß Anspruch 1, dadurch gekennzeichnet, daß darin

$R^1$ für Wasserstoff, Fluor, Chlor, Brom, Methyl, Ethyl, Trifluormethyl, Chlormethyl, Methoxymethyl, Methoxy, Ethoxy, Difluormethoxy, 2,2,2-Trifluorethoxy, 2-Methoxy-ethoxy, Methylthio, Methylamino, Ethylamino oder Dimethylamino steht,

$R^2$ für Wasserstoff, Fluor, Chlor, Brom, Methyl, Trifluormethyl oder Methoxy steht,

$R^3$ für Wasserstoff, Fluor, Chlor, Brom, Methyl, Ethyl, Trifluormethyl, Chlormethyl, Methoxy, Ethoxy oder Difluormethoxy steht,

$R^4$ für Wasserstoff, Methyl, Ethyl, Propyl, Isopropyl, Butyl, Isobutyl, sec-Butyl, tert-Butyl, Allyl,

Propargyl, Phenyl oder Benzyl steht,

$R^5$ für Wasserstoff steht und

$R^6$ für den Rest

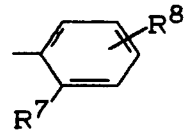

steht, worin

$R^7$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, N-Methoxy-N-methylaminosulfonyl, Phenyl, Phenoxy oder $C_1$-$C_3$-Alkoxy-carbonyl steht und

$R^8$ für Wasserstoff, Fluor oder Chlor steht;

weiter

$R^6$ für den Rest

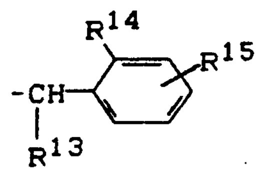

steht, worin

$R^{13}$ für Wasserstoff steht,

$R^{14}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{15}$ für Wasserstoff, Fluor oder Chlor steht;

weiter

$R^6$ für den Rest

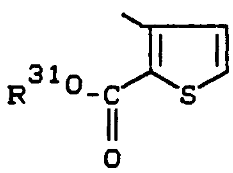

steht, worin

$R^{31}$ für Methyl oder Ethyl steht, oder

$R^6$ für den Rest

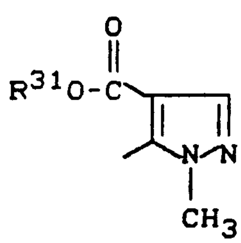

steht, worin

$R^{31}$ für Methyl oder Ethyl steht.

**4.** Verfahren zur Herstellung von substituierten Sulfonylaminotriazolylpyrimidinen der allgemeinen Formel (I) gemäß Anspruch 1, dadurch gekennzeichnet, daß man Thiosemicarbazid-Derivate der Formel (II),

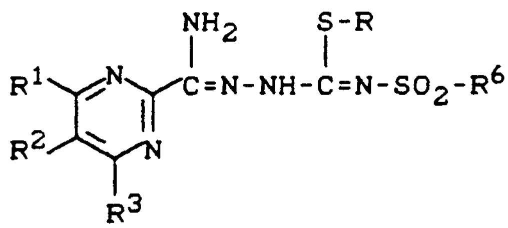

(II)

in welcher

$R^1$, $R^2$, $R^3$ und $R^6$ die in Anspruch 1 angegebene Bedeutung haben und

R für Alkyl steht,

und/oder Tautomere der Verbindungen der Formel (II) mit Aminen der allgemeinen Formel (III)

$H_2N\text{-}R^4$

in welcher

$R^4$ die in Anspruch 1 angegebene Bedeutung hat,

gegebenenfalls in Gegenwart eines Verdünnungsmittels umsetzt und gegebenenfalls die so erhaltenen Verbindungen der Formel (I), in welcher $R^5$ für Wasserstoff steht, durch Umsetzung mit Ammoniak, Alkyl-, Dialkyl- oder Trialkylaminen oder mit geeigneten Metallverbindungen nach üblichen Methoden in entsprechende Salze überführt.

**5.** Herbizide Mittel, gekennzeichnet durch einen Gehalt an mindestens einer Verbindung der Formel (I) gemäß Anspruch 1.

**6.** Verfahren zur Bekämpfung von Unkraut, dadurch gekennzeichnet, daß man eine Verbindung der Formel (I) gemäß Anspruch 1 auf die Unkräuter oder ihren Lebensraum einwirken läßt.

**7.** Verwendung von Verbindungen der Formel (I) gemäß Anspruch 1 zur Bekämpfung von unerwünschtem Pflanzenwachstum.

**8.** Verfahren zur Herstellung von herbiziden Mitteln, dadurch gekennzeichnet, daß man Verbindungen der Formel (I) gemäß Anspruch 1 mit Streckmitteln und/oder oberflächenaktiven Mitteln vermischt.

**9.** Thiosemicarbazid-Derivate der allgemeinen Formel (II),

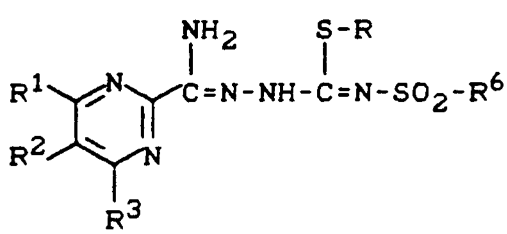

(II)

in welcher

R für Alkyl steht,

$R^1$, $R^2$ und $R^3$ gleich oder verschieden sind und für Wasserstoff, Halogen, Alkyl, Halogenalkyl,

Alkoxyalkyl, Alkoxy, Halogenalkoxy, Alkoxyalkoxy, Alkylthio, Alkylamino oder Dialkylamino stehen, wobei zwei dieser Reste $R^1$ und $R^2$ oder $R^2$ und $R^3$ auch zusammen für Alkandiyl stehen können, und

$R^6$ für jeweils gegebenenfalls substituiertes Aralkyl, Aryl oder Heteroaryl steht.

Europäisches Patentamt

# EUROPÄISCHER RECHERCHENBERICHT

Nummer der Anmeldung

EP 91 11 8130

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | DE-A-3 737 748 (SCHERING AG)<br>* Ansprüche 1,4-6 * | 1,5-8 | C07D403/04<br>A01N43/653<br>//(C07D403/04,<br>249:00,239:00) |
| D,Y | DE-A-3 825 867 (BAYER AG)<br>* Seite 48, Verbindung (IA-2); Seite 87, Verbindungen (IC-224), (IA-209) *<br>* Ansprüche 1,4-7 * | 1,5-8 | |
| A,P | EP-A-0 419 831 (BAYER AG)<br>* Ansprüche 1,3,5-10 * | 1,5-8 | |
| A | EP-A-0 338 465 (SCHERING AG)<br>* Ansprüche 1,3,4,6 * | 1,5,7,8 | |
| A | EP-A-0 246 749 (SCHERING AGROCHEMICALS LTD.)<br>* Ansprüche 1,10 * | 1,5,7 | |
| A,D | JOURNAL OF ORGANIC CHEMISTRY.<br>Bd. 30, Nr. 3, März 1965, EASTON US<br>Seiten 931 - 933;<br>F.H. CASE: 'The Preparation of Hydrazidines and as-Triazines Related to Substituted 2-Cyanopyridines'<br>* Seite 932, Tabelle I * | 4,9 | **RECHERCHIERTE SACHGEBIETE (Int. Cl.5 )**<br>C07D |

Der vorliegende Recherchenbericht wurde für alle Patentansprüche erstellt

| Recherchenort | Abschlußdatum der Recherche | Prüfer |
|---|---|---|
| BERLIN | 17 FEBRUAR 1992 | CHRISTIAN HASS |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument